# EUROPEAN PATENT SPECIFICATION

(11) **EP 1 725 163 B1**
(45) Date of publication and mention of the grant of the patent: **08.12.2010**
(21) Application number: 05718033.3
(22) Date of filing: 15.03.2005
(51) Int. Cl.: A61B 5/00, A61B 5/087, G06F 19/00

(54) **MEDICAL DATA DISPLAY**
MEDIZINISCHE DATENANZEIGE
DISPOSITIF D'AFFICHAGE DE DONNEES MEDICALES

(30) Priority: 15.03.2004 GB 0405798
(43) Date of publication of application: 29.11.2006
(60) Divisional application: 09009628.0
(73) Proprietor: E-San Limited, Oxford 0X4 4GA (GB)
(72) Inventor: TARASSENKO, Lionel, University of Oxford, Oxford OX1 3PJ (GB); HAYTON, Paul Michael, e-San Limited, Oxford OX4 4GA (GB); GEORGE, Alastair William, e-San Limited, Oxford OX4 4GA (GB)
(74) Representative: Nicholls, Michael John
(86) International application number: PCT/GB2005/000980
(87) International publication number: WO 2005/087091

(56) References cited:
- EP-A- 1 364 666
- US-A- 5 732 709
- US-A- 5 812 983
- US-A- 5 827 180
- US-A1- 2001 034 478
- US-A1- 2003 055 323
- US-A1- 2003 172 925
- US-A1- 2003 208 113
- US-A1- 2004 034 295
- US-B1- 6 283 923

## Description

The present invention relates to a method and apparatus for displaying medical data, particularly data indicative of the current state of a chronic medical condition in a form which can be delivered easily to a patient and is easily understandable by them.

As many as 20% of the population in the Western world suffer from chronic medical conditions such as asthma, diabetes or hypertension. The management of these conditions represents a significant drain on healthcare budgets and, of course, the conditions themselves cause distress and inconvenience for the sufferers. Traditionally the management of the patient suffering from a chronic condition such as these involves requiring the patient to attend regular medical clinics where they consult with a clinician and on the basis of measurements and observations made by the patients themselves in between the clinics, and on the basis of measurements made at the clinics, the patient's medication can be assessed and adjusted if necessary. A significant amount of self-management and self-discipline is therefore required of the patients in the period between clinics in that they must administer medication to themselves appropriately and must try to observe and record their symptoms correctly.

For example, asthma is a chronic condition which requires self-management by patients. In this case the self-management is by use of an inhaler administering a pharmacological agent for management of the condition, for example by administration of a drug such as a steroid in powdered form by using an inhaler. Between regular clinics at which the patient's condition is discussed, the patient is required to keep a patient diary in which they record their symptoms, their use of the inhaler, and in which they also record measurements of their condition taken using a device such as a respiratory flow meter for measuring their Peak Expired Flow Rate (PEFR) and forced expired volume (FEV1-integrated volume of air exhaled over the first second). Cystic fibrosis sufferers also make similar measurements of their condition.

Other chronic conditions such as Chronic Obstructive Pulmonary Disease (COPD) and hypertension require similar self-management by the patient and regular visits to a clinic or doctor's surgery.

Another example of a chronic condition which requires self-management is diabetes. Diabetes sufferers have to manage their diet and exercise and if they are insulin-dependent (Type 1 diabetes) or insulin-treated, administer insulin themselves, usually four times a day for Type 1 diabetes, recording the dosages and their symptoms and also measuring their blood glucose levels by taking a small blood sample and using a proprietary test device. At the regular clinics the recordal of these activities and the patient diary is monitored, and also a measurement is made of their glycosylated haemoglobin (HbAl_{c}) which gives an indication of the average blood sugar levels over the last three months. While the managing of diabetes from day-to-day is inconvenient for the patient, mis-management of the condition also has long-term consequences for the patient's health. From day-to-day it is important that the patient does not become hypoglycaemic (blood sugar level too low - leading to confusion and fainting), but for the long-term health of the patient it is important that they are not hyperglycaemic (blood sugar level generally too high) because this gives rise to long-term kidney problems, nerve damage, poor circulation, blindness and a high risk of stroke. Thus it would be advantageous to be able to help the patient visualise their day-to-day blood glucose levels.

For a number of years proposals have been made for so-called "e-health" systems which are variously designed to provide technological solutions for allowing the patient to measure healthcare data (such as peak flows, blood glucose or blood pressure) and record their symptoms, and possibly communicate the recorded data to a clinic. Early proposals based on the use of a telephone for self-reporting with readings transferred onto a home computer by the patient, or a monitoring device connected to a modem for data transmission have not proved in practice to be useful. In such systems data transfer usually occurs once a day at most, and sometimes as infrequently as once a week. The systems generally require the clinicians to review all of the data once a day, or even once a week, but this is time-consuming and bothersome for the clinicians. Furthermore, patients often find it troublesome to transfer readings onto a home computer or Personal Digital Assistant (PDA) and to obtain a suitable connection for transferring the data. This has led to a lack of long-term use of these systems.

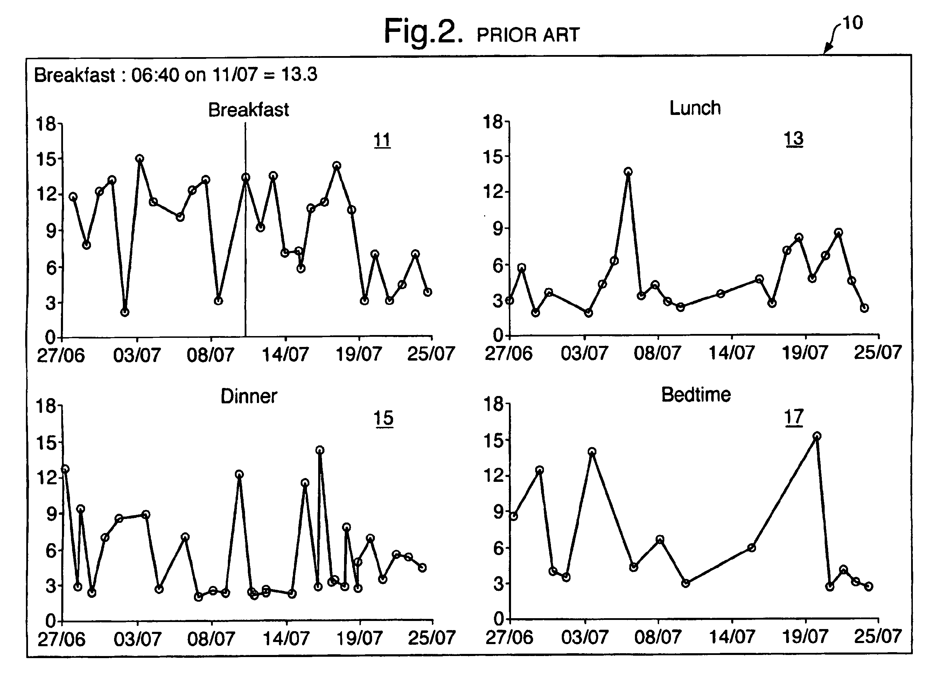

Also, the usefulness of the data presented to patients is limited. In the case of electronic recordal for a diabetes sufferer for example, the most that might be available to a patient is recordal and display of blood glucose levels from day-to-day, for example as illustrated in Figure 2 of the accompanying drawings. In the display 10 of Figure 2, the blood glucose levels from day-to-day as measured at four different times of day, namely breakfast, lunch, dinner and bedtime, are indicated in the plots 11, 13, 15 and 17. It can be seen that there is a large variation, but there is no indication of whether this represents good or bad control of their diabetes for that patient.

It has also been found that there can be a high amount of random variability in the patient's condition with self-management regimes. This may be due to external factors affecting the patient, or to variation in the patient's actions under the regime, For example, asthma is weather and season-dependent (external factors), and the peak flow reading taken by the patient depends on how recently they used their inhaler - e.g. sometimes they use it before the reading and sometimes after. Patients often cannot remember the correct action sequence. Also they may not remember the appropriate action if their readings are abnormal.

The present applicants have proposed a more convenient system as described in WO 2004/027676, in which a measurement device such as a peak flow meter or blood glucose meter can be connected directly to a GPRS or 2.5 or 3G mobile telephone, (sometimes known as a smart phone), adapted so as automatically to receive the medical data from the measurement and transmit it without patient intervention to a remote server. The data is processed at the remote server and a reply sent immediately to the patient. This provides significant advantages over earlier systems because it requires little work by the patient, for example no intermediate paper records need to be prepared, the interaction with the telephone is quick and easy because the data is transmitted in real time automatically, and there is immediate clinical feedback to the patient from the remote server. Also, because most of the data generated by patients with chronic conditions are normal, the system can involve the clinician only when readings become abnormal. Therefore it reduces the workload on the clinician as compared with previous proposals. Modem fashion has also resulted in mobile telephones becoming a part of many people's lifestyle and the use of the mobile telephone for healthcare can build on this, resulting in improved self-management. This can improve the control of the patient's condition and their long-term health, and thus have significant benefits for the health of the population.

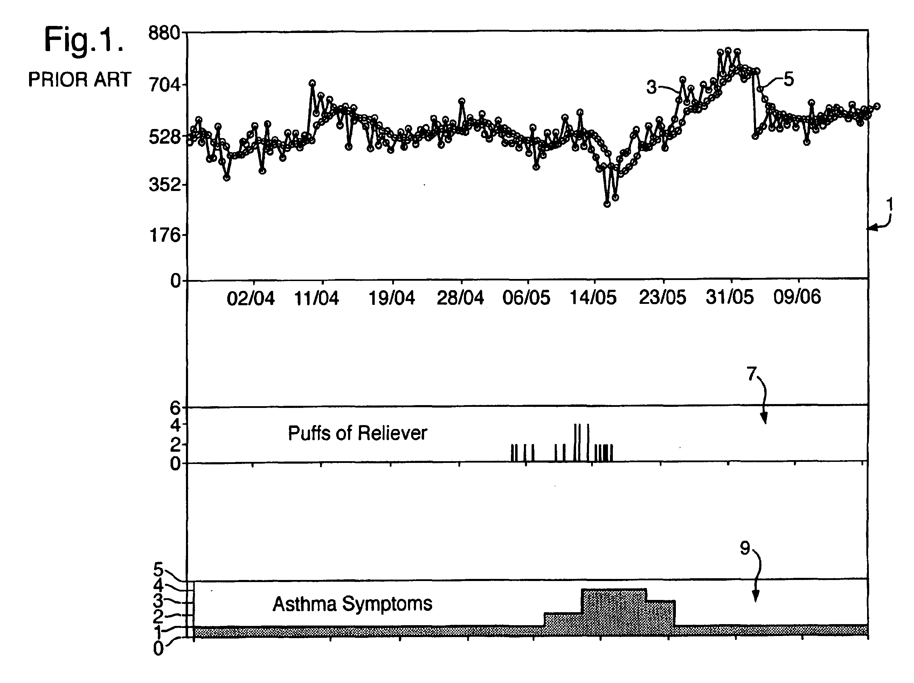

Figure 1 illustrates a display of data recorded for an asthma sufferer in the above system. In an upper part of the display 1 there is a plot of the daily peak flow readings 3, together with a trend 5 calculated from those daily readings. In the lower part of the display 1 a plot based on the patient's recorded use of the inhaler 7 and a qualitative assessment by the patient of the seriousness of their own symptoms 9.

An important.part of encouraging patients to improve self-management, though, is to provide the medical data to them in a convenient and easily understandable way. In particular the limitations on screen size in portable electronic devices such as mobile telephones, make this especially difficult. A display such as that shown in Figure 1 cannot easily be fitted onto a small screen. Displays such as that shown in Figure 2 are difficult to understand and do not give any indication of how the use of the pharmacological agent (insulin) is controlling the medical condition.

US-A1-2003/0055323 discloses a portable automatic insulin syringe device with blood sugar measuring function. It records and displays insulin administrations and regularly measures, records and displays the patient's blood sugar level. Both the amount of insulin administered and the blood sugar measurements are displayed on the same rolling 24 hour plot.

There is therefore a need for improved displays of medical data, which give easier indications of the readings taken and of their significance.

Accordingly the invention provides a method of displaying data indicative of the control of a medical condition by administration of a pharmacologically active agent, as defined in claim 1.

This aspect of the invention is particularly useful in forming an educational tool whereby the quality of control of the condition by use of the pharmacological agent can be easily seen over a period of time.

The visual link can comprise colour coding of the plotted values and, to assist understanding, the time axes in the two display areas are preferably aligned. The predetermined time period may be a day and the corresponding pairs from each set may be pairs that temporally correspond, for example being at the same time of day, or being related to the same respective activities during the day (such as the same meals and bedtime).

This aspect of the invention also provides apparatus for displaying such data in this way

In the invention the processing of the data for display and the control of the display itself may be embodied in suitable software which runs as an executable application on an electronic device, and optionally at a remote server too. Thus it may run as an application on a mobile telephone or PDA or other portable electronic device. The invention therefore extends to a computer program which when loaded on a suitable device executes the display according to the invention.

The invention will be further described by way of example with reference to the accompanying drawings in which:-
Figure 1 illustrates a prior art display of an asthma sufferer's condition;
Figure 2 illustrates a prior art display of blood glucose measurements for diabetes management;
Figures 3(A) and (B) illustrate displaying data relating to an asthma sufferer's condition;
Figures 4(A), (B) and (C) illustrate displaying data related to blood glucose control of a diabetes sufferer;
Figure 5 illustrates displaying further data related to a diabetes sufferer's condition;
Figure 6 illustrates an embodiment of the invention, also relating to a diabetes sufferer's condition management; and
Figures 7A to 7L illustrate a sequence of displays.

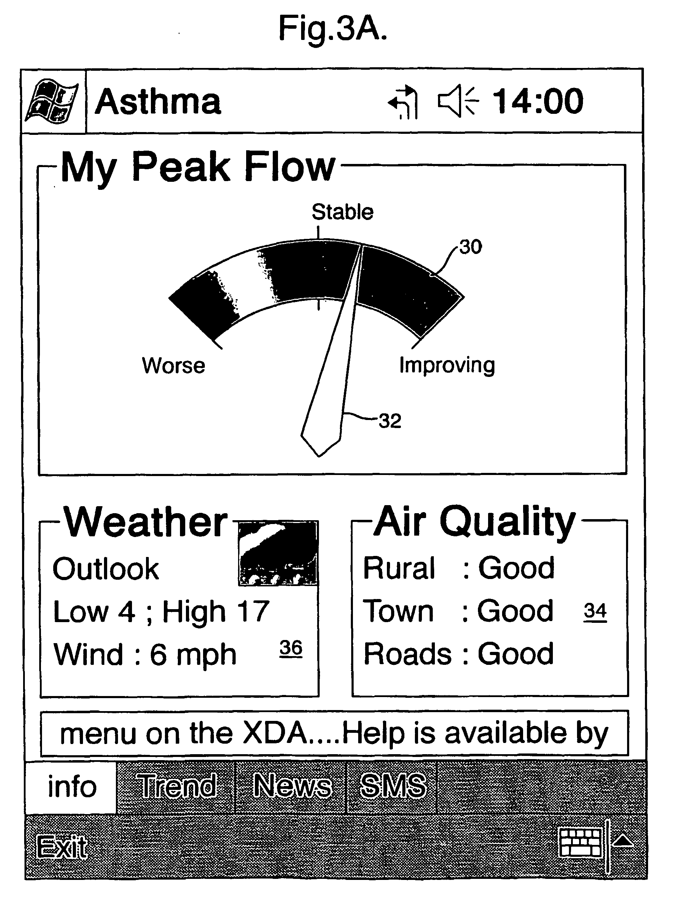

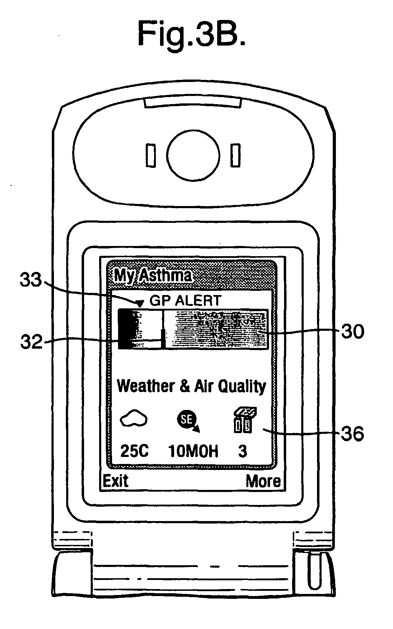

Figures 3(A) and (B) illustrate alternative versions of a display for use by an asthma sufferer. The display consists of a first graphical element 30 in the form of a scale colour-coded from red at the left-hand side through amber and yellow to green at the right-hand side. Figure 3(A) shows an arcuate version of the scale and Figure 3(B) a straight version. As will be explained below, the scale is not fixed but is based on a model of normality for the particular patient. It therefore differs from a traditional fixed scale or a representation of such a fixed scale. The display includes a second graphical element 32, in this case in the form of a needle, which is used to indicate the current, i.e. today's, condition of the patient. This display is based on indicating to the patient a peak flow reading as obtained from a peak flow meter.

The second graphical element 32, the needle, will be displayed pointing to a position on the scale representing the current peak flow value. Normally a peak flow reading is taken by the patient conducting three measurements in quick succession, i.e. blowing into the peak flow meter three times in succession, and then the average of the three readings can be taken, or the best of the three can be taken. This becomes the current reading and it is this which the needle displays.

The first graphical element, namely the scale 30, is a model of normality for the patient which is based on calculation of a trend of relatively recent peak flow readings. The trend may be calculated during an initial learning period (for example a month) in which the best peak flow readings (excluding outliers) are used to set the 100% value. It can also be made adaptive by using a Kalman filter to reflect the long-term trend (such as 3 or 6 months). The scale is then calculated and displayed with the green (right-hand end) set at 100% of the best peak flow value, the green to amber transition at 75% of this value, the amber to red transition at 50% of this value. A GP alert value at 75% may be indicated on the scale as shown by label 33 in Figure 3(B), being the value at which a message is sent automatically to a clinician alerting them to a significant worsening in patient condition. Of course, different percentage values may be chosen. To initialise the device a learning set of 30 days readings may be used, or standard default values, these being replaced as the Kalman filtered trend values resulting from normal daily readings become available.

The model may also include as a parameter a score based on the patient's own assessment of condition, taken from a patient diary as explained below.

Comparing this display to the top plot in Figure 1, therefore, it can be seen that as the trend 5 varies, the actual value represented by the colour background may vary. This corresponds to the patient-specific model of normality varying with time. For example, in the Spring or Summer or when air pollution is bad, causing symptoms generally to be bad, the 100% position on the scale may represent a much lower peak flow reading than at a time when the patient's condition is generally good.

In the display described above the scale is set according to the model of normality for measurements on that patient. Alternatively, however, the scale can be judged from standard data suitable for that patient judged from the population. Thus the scale would differ according to the sex, weight, age and so on of the patient.

Figures 3(A) and (B) illustrate a further feature in that the display includes information relevant to the patient's condition such as weather 36 and air quality 34. This can be adapted to the location of the patient using the GPS data available in GPRS telephones, or the cell data in a normal cellular telephone system. The availability of this data allows it to be incorporated into the model if desired. For example, a greater degree of variability in the patient's condition can be expected in cold weather or if the pollen count is high. The model can take this into account and enlarge the scale shown in anticipation of higher variability.

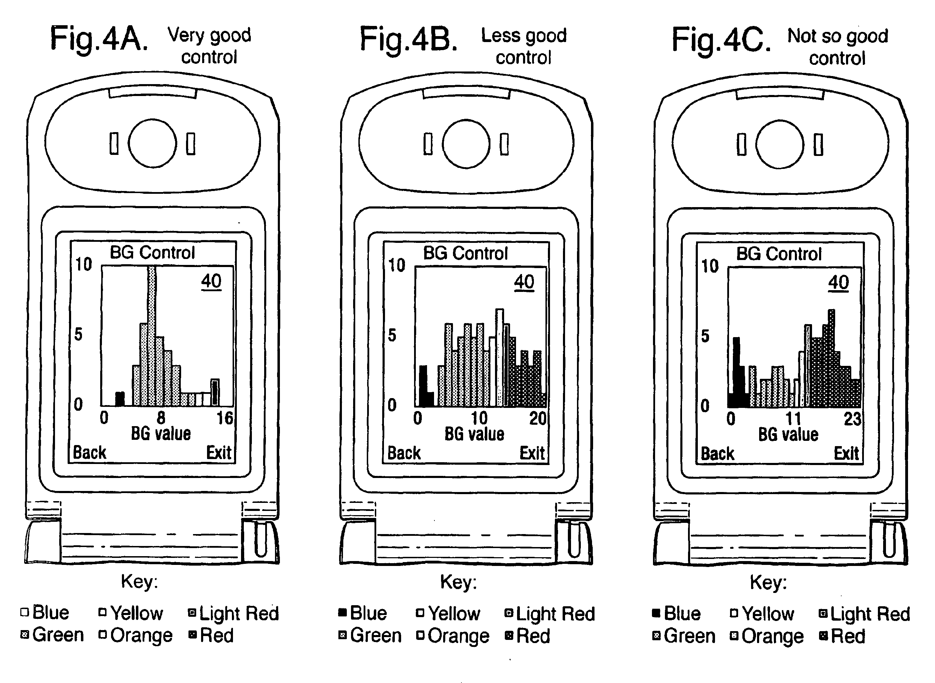

Figures 4(A) to (C) illustrate a display which is useful for diabetes sufferers. In Figures 4(A), (B) and (C) the display shows a histogram 40 of blood glucose measurements taken by the patient. Thus the blood glucose value is plotted along the horizontal axis, with the frequency of occurrence of that value plotted vertically. The histogram contains the most recent 2 weeks worth of readings (usually 56 readings at 4 readings per day). The horizontal axis is autoscaled so as to show the full range of readings obtained. Thus a patient with good blood glucose control will tend to see a smaller range of values on the horizontal axis (0-16 in Figure 4A) than a patient with less good control (0-20 in Figure 4B) or poor control (0-23 in Figure 4C). The histogram is also colour-coded according to a patient specific model of normality, in this case comprising thresholds for hypo and hyperglycemia for that patient. These target thresholds are set by a clinician by agreement with the patient on the basis of the patient's history of blood glucose control.

Thus a transition from green to blue (indicating hypoglycemia) may be set normally somewhere between BG values of 3 and 7. The transition from green to red (indicating hyperglycemia) may be set normally somewhere between 9 and 16. These values may be set at a clinic by a clinician, and a new patient with poor control may have the target thresholds set more widely than a patient with experience - who has demonstrate better control.

A patient, therefore, whose blood sugar level is not being controlled properly may see a range from 0 to 25, most of which will be red. As their condition becomes better controlled (e.g. they become better at controlling diet or judging insulin dose), the range displayed will gradually narrow down until it is from 0 to 20 or 0 to 16 as illustrated, when green will be in the middle and most readings, and thus most of the histogram, will be green. So viewing the histogram gives an immediate indication of current condition.

At a clinic, the target thresholds may be adjusted (e.g. the hyperglycaemic target threshold reduced); so that the colours on the histogram reflect the model of normality for that patient.

As mentioned above, one of the problems in self management plans is that patients may not follow rigorously the actions prescribed for them. Further, they may forget the details of their self management plan. This allows the agreed healthcare plan to be stored on the patient's device (e.g. telephone) so that the patient can refer to it at any time. The plan can be updated by automatic updates from the server. Further, the clinician can include a reminder to be displayed in response to certain values of the measurements made by the patient. For example, if the patient's condition deteriorates, they can be reminded what action to take, such as increase the use of the reliever/inhaler in the case of asthma, or if the patient's condition becomes dangerous, they can be reminded what emergency action to take, such as contacting a healthcare professional and/or administering an emergency dose of their medicament.

The variability in the following of the plan by the patient can be reduced by providing for the display to guide the patient with a particular workflow. This workflow should follow a "measurement-evaluate-act" sequence so that the patient first measures their condition, then this measurement is evaluated (with the assistance of the automatic processing and display of the data) and then the appropriate action is taken, again with the assistance of the agreed plan accessed on the display.

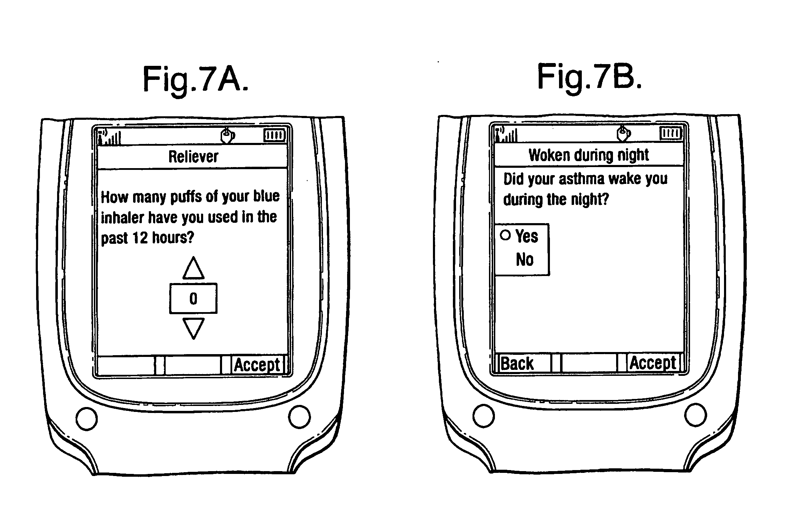

An example workflow, for the asthma monitoring device, is as follows:-
1) The patient starts the application on their telephone.
2) The patient is presented with a screen such as that shown in Fig. 7(A) asking how many puffs of reliever of inhaler he/she has used during the last 12 hours.
3) The patient is then asked to grade their asthma symptoms using three questions:
   (i) *"did your asthma wake you during the night*/*last night";*
   (ii) *"did you have your usual asthma symptoms today*/*yesterday?";*
   (iii) *"did your asthma stop any of your usual activities in the last 24 hours?".*

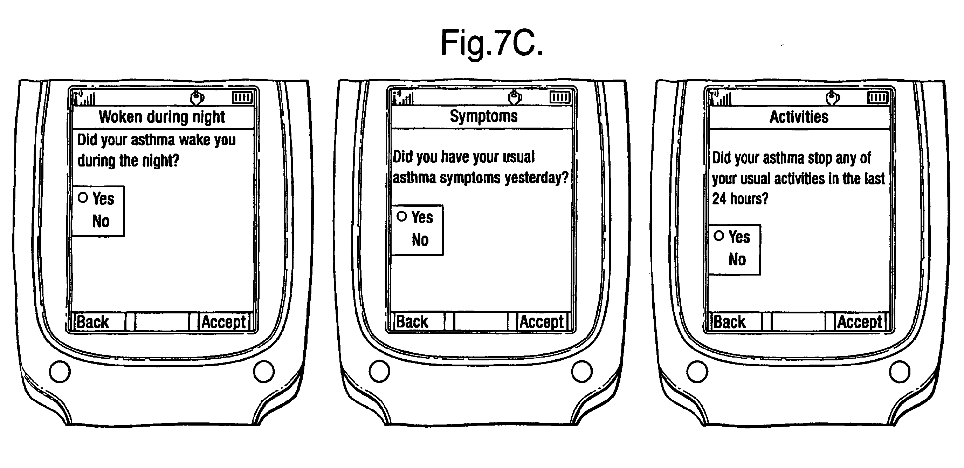

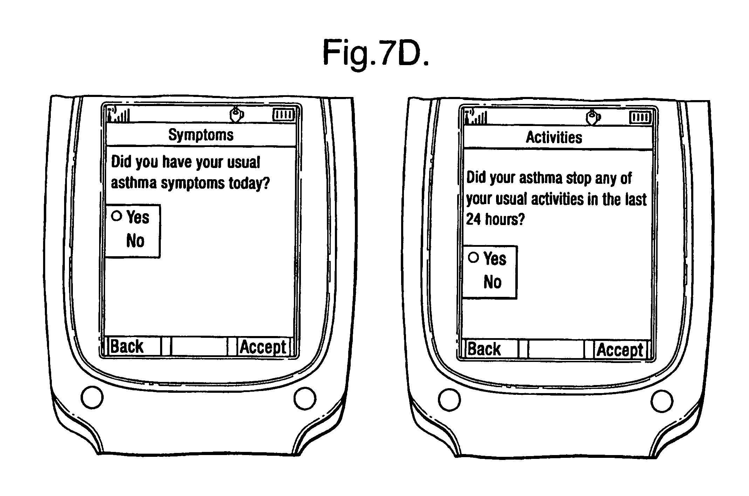

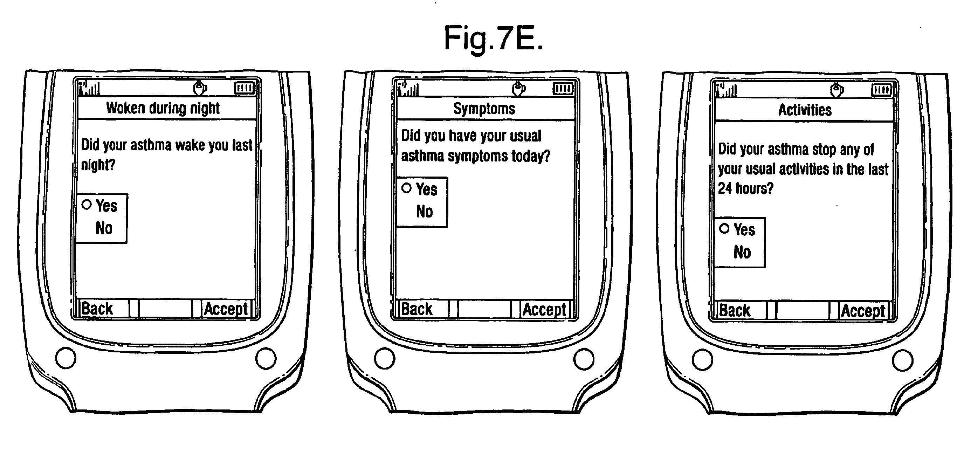

   The questions are tailored depending on the time of day and the previous entries by the patient. For example, depending on whether the patient uses the application both in the morning or evening, or just once in a 24 hour period, they will be asked one, two or three questions. In fact, there are four cases, namely:
   Case 1 - in which the patient is taking a reading in the morning and has taken one the previous evening (in this case the display of Fig. 7B only is shown);
   Case 2 - in which the patient is taking a reading in the morning and has not taken one the previous evening (in which case the three questions as illustrated in Fig. 7C are displayed in sequence);
   Case 3 - in which the patient is taking a reading in the evening and has taken one that morning (in which case the two questions displayed in Fig. 7D are displayed in sequence); and
   Case 4 - in which the patient is taking a reading in the evening and has not taken one that morning (in which case the three questions shown in Fig. 7E are displayed in sequence).

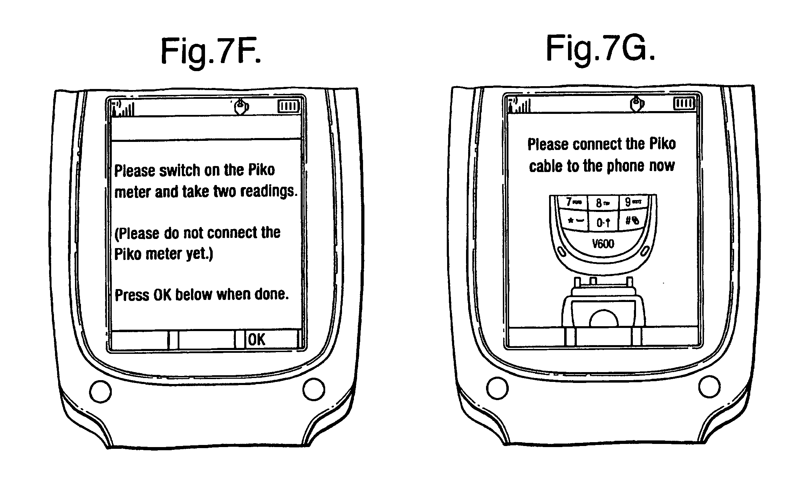

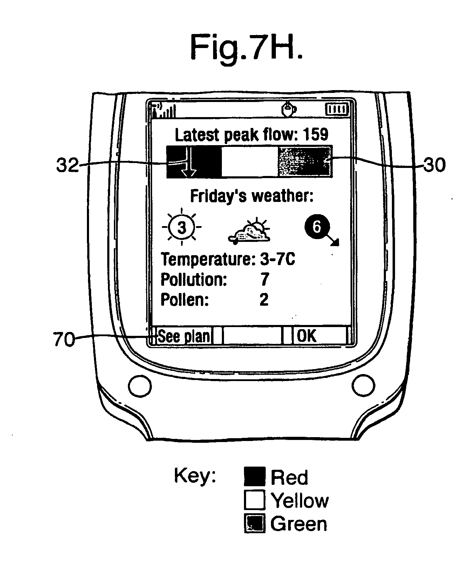

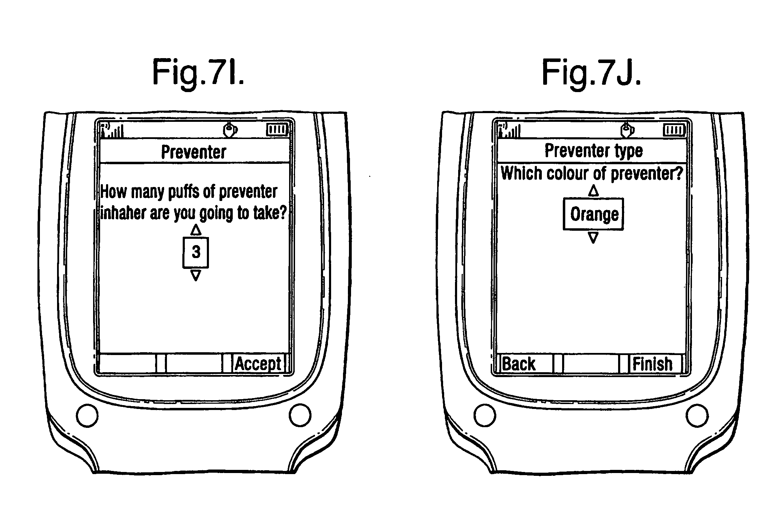

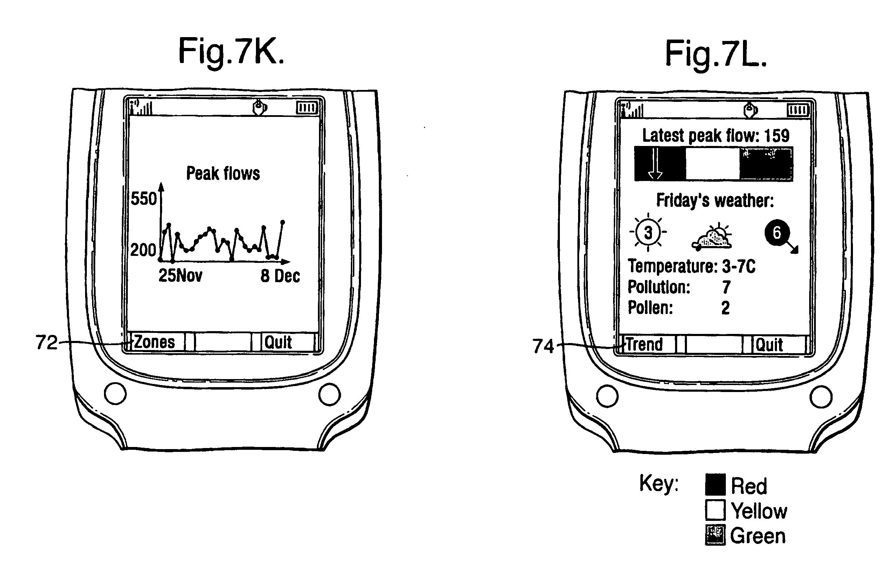

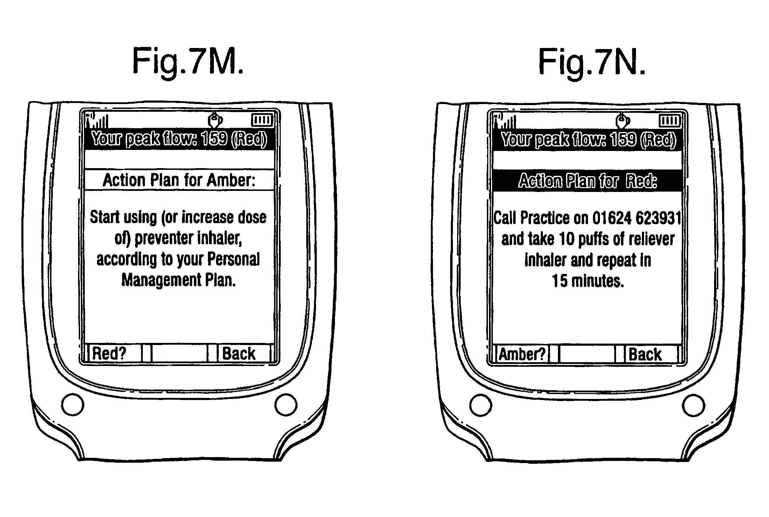

   The answers to these questions can be used to give a score of the severity of the symptoms. This score can be displayed separately, for example as shown at 9 in Fig. 1, or can be used to adjust the model of normality for the patient, or can be displayed in a similar manner to the peak flows as shown by 30 and 32 in Figure 3A.
4) The patient is asked to switch on the peak flow meter and take an appropriate number of readings as shown by the display of Fig. 7F.
5) The patient is then asked to connect the peak flow meter to the telephone as shown by the display in Fig. 7G. This connection can be automatical and wireless using for example the "Bluetooth" ® protocol.
6) A personalised display of the patient's condition is shown, together with local external conditions such as weather and air quality, as shown in Fig. 7H.
7) The display of Fig. 7H includes a button 70 which allows the patient to access their agreed treatment plan. For example, an appropriate part of the agreed treatment plan may be displayed in dependence upon the current measurement of the patient's condition. Fig. 7M shows an example of the treatment plan in the case that the patient's condition is in a warning zone, and in this example recommends an increased dosage of medicament. Fig. 7N illustrates an example of a part of the treatment plan appropriate for a reading showing that the patient's condition is dangerous, in this case an emergency administration of medicament together with a recommendation to contact a clinician.
   On exiting the treatment plan, the patient is then asked to input what action they are going to take by means of the displays shown in Figs. 7I and 7J. In this case this involves indicating how many puffs of preventor inhaler will be administered and of which type.
8) The readings or the best reading from the patient's measurement of their condition in step 4 is then transmitted to the server.
9) Finally, the feedback screens of Figs. 7K and 7L are shown interchangeably. In Fig. 7K the trend of recent readings is illustrated but this display can be changed to the personalised display of Fig. 7L using the "zones" button 72. The trend may be accessed from the personalised display of Fig. 7L by using the "trend" button 74.

The patient-specific model of normality is maintained on the patient's device (e.g. telephone) so that in the event of a loss of connectivity to the server, at least the personalised display of Fig. 7L can be shown, possibly absent the local condition (weather) data which is delivered from the server. The patient's readings are stored for later transmission to the server when connectivity is restored.

It can be seen that the workflow provided by the display encourages the patient to use the measure-evaluate-act sequence which assists in a more consistent assessment and control of the patient's condition.

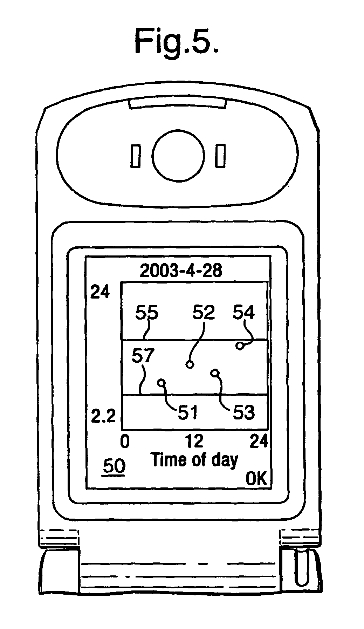

Figure 5 illustrates another display 50 useful for diabetes sufferers. In this case the patient has taken four blood sugar measurements through the day which are labeled 51, 52, 53 and 54 and these are plotted against time of day on the horizontal axis and blood sugar level on the vertical axis. However, the display also illustrates two target thresholds 55 and 57 which represent the limits of acceptable blood glucose level for this patient as discussed above. Thus 57 is the lower acceptable value of blood glucose (the green to blue transition above) and 55 is the upper acceptable value (the green to red transition above). The lower value is set to avoid hypoglycaemia. The upper level is the current target threshold for hyperglycaemia agreed with that patient. An advantage of displaying the upper threshold (and the red area in the histogram) is that the patient knows that if many of their daily blood glucose measurements are near or exceed the level on the display at which the upper limit 55 is set, their glycosylated haemoglobin (HbAlc) is likely to rise over time above the accepted level.

Because in Figures 3(A), 3(B), 4(A) to (C) and 5 the display shows a model of normality which is tailored to the specific patient, the best use can be made of a limited display area in showing a patient data which is relevant to him or her, without needing to allow, in the display, area for showing data suitable for all patients.

Thus it is suitable for the compact displays found on portable electronic devices such as PDAs and mobile telephones, though the display is not, of course, limited to this.

In the case of a telemedicine system the readings taken at the patient end may be transmitted to a server and a response sent to the patient which includes the patient-specific model of normality (the server storing a model for each patient). Thus the background of the display may be based on data at the server, while the current value is based on the reading at the patient end. The new readings are added to the data for that patient at the server, of course, and may be used to adapt the model of normality dynamically (e.g. in the display of Figures 3(A) and (B) by contributing to the trend calculation).

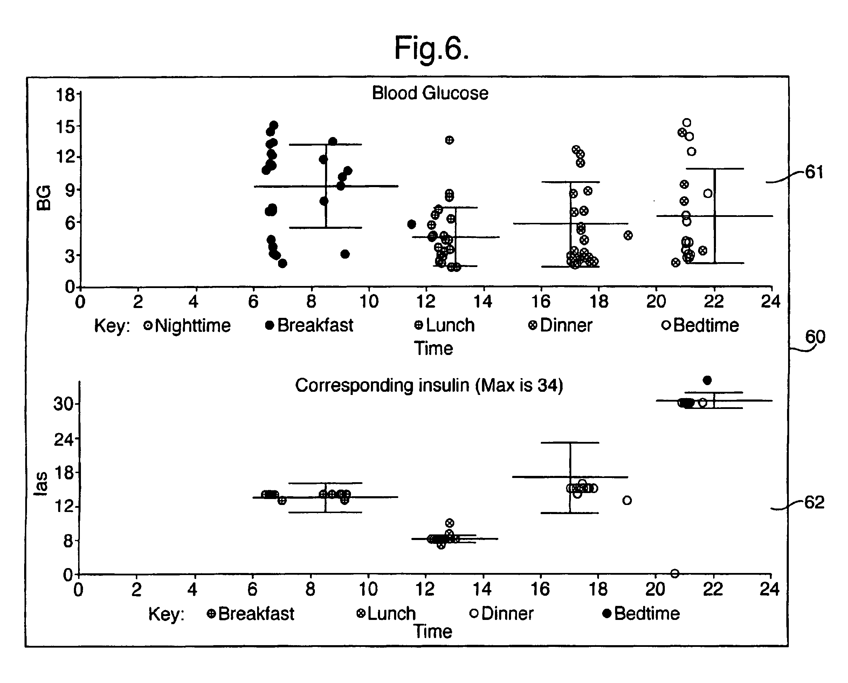

Figure 6 illustrates an embodiment of the invention which is useful as an educational tool for helping diabetes patients improve their control of their condition by adjusting their insulin dosage. As shown in Figure 6 the display 60 has an upper pane 61 and a lower pane 62. In the upper pane 61 a horizontal scale corresponding to a single twenty four hour day is shown. The vertical scale plots blood glucose level. Each of the blood glucose readings taken by the patient over a period of many days (four weeks in Figure 6) is plotted on the same plot at the appropriate point for the time of day and blood glucose level. Furthermore, all of the readings which correspond to the same time of day are colour-coded. For example, all of the readings at breakfast time are coloured red, at lunchtime coloured blue, at dinnertime coloured green and at bedtime coloured purple. It will be seen that some of the dinnertime readings are at a time of day which, on other days, has corresponded to bedtime. This is normal and the readings can be characterised as dinner or bedtime by requiring the patient to indicate which it is when entering the data, or by judging whether it is the second, third, fourth or fifth reading of the day and the time of day of generation of the reading.

The display also includes a lower pane 62 which plots insulin dosage as input by the patient. The dosage of insulin is plotted vertically and, again, the horizontal axis represents a single 24 hour period. The insulin dosages for each day over the same period are plotted, and are again colour-coded according to the time of administration, namely breakfast, lunch, dinner or bedtime. It will be seen by the colour-coding that the insulin administered at breakfast time controls the blood glucose level as measured at lunchtime. The insulin administered at lunchtime controls the blood glucose levels at dinnertime. That at dinnertime controls the level at bedtime and that administered at bedtime (usually a much higher administration to last through the night) controls the level of blood sugar as measured the next morning. The use of the colour-coding as a visual link between the two plots makes it simple for the patient to see the connection between the insulin administration and the corresponding control of blood glucose.

To improve the patient's condition the aim is to keep the blood glucose level stable, and thus to avoid large vertical scatter of blood glucose level in each group. Figure 6 illustrates a rather large scatter of blood glucose level in each group, thus indicating poor control. It can be seen that the insulin dosage is very stable (the scatter in dosage in each of the insulin administration groups is small). Thus in the illustrated case the patient can easily see that they are not varying the insulin dosage sufficiently to control the blood glucose level stably. A patient who is better at control would have a larger scatter of insulin dosages (i.e. the groups in the lower plot would be more spread out vertically), and have much tighter vertically distributed groups in the upper plots 61.

Therefore the combination of using a visual link, in this case colour coding, between the measurement of the parameter representing patient's condition (blood glucose level) and the administration of pharmacological agent to control that condition (insulin), together with the plotting of several days of data on the same plot make a good educational tool in which the patient can easily see whether they are successfully controlling their condition.

## Claims

1. A method of displaying data indicative of the control of a medical condition over several successive different time periods by administration of a pharmacologically active agent, comprising:-
acquiring as said data a plurality of sets of measured values of a parameter indicative of said medical condition, each set comprising a plurality of measured values of said parameter during each successive different predetermined time period, and a plurality of sets of values of administration of said pharmacologically active agent, each set comprising a plurality of administration values during each successive different predetermined time period;
displaying in a first display area (61) a plot of the measured values of the parameter from all of said sets against a time axis;
displaying in a second, adjacent display area (62) a time plot of the values of administrations of said pharmacologically active agent from all of said sets against a time axis;
said first and second display areas (61, 62) each displaying a single representative time period equal in length to one of said successive different predetermined time periods, all of the administration and measured values from said successive different time periods being plotted in said single representative time period; and
displaying a visual link between each pair of displayed values formed by an administration value and a measured value of the parameter corresponding to response of the medical condition to that administration; and wherein
said visual link is: (a) common for corresponding pairs from each of the sets; and (b) visually-distinguishes the different pairs in each set from each other.

2. A method according to claim 1 wherein the visual link comprises colour coding of said plotted values.

3. A method according to claim 1 or 2 wherein the time axes in the two display areas are aligned.

4. A method according to claim 1, 2 or 3 wherein the predetermined time period is a day.

5. A method according to claim 4 wherein the corresponding pairs from each set are pairs that temporally correspond.

6. A method according to claim 4 or 5 wherein the corresponding pairs from each set are pairs that are at the same time of day.

7. A method according to claim 4 or 5 wherein the corresponding pairs from each set are pairs that correspond to the same respective activities during each of the days.

8. A method according to claim 4 or 5 wherein the corresponding pairs from each set are pairs that correspond to the same meals and to bedtime during each of the days.

9. Apparatus for displaying data indicative of the control of a medical condition over several successive different time periods by administration of a pharmacologically active agent, comprising:-
a display (60);
a data acquisition device for acquiring as said data a plurality of sets of measured values of a parameter indicative of said medical condition, each set comprising a plurality of measured values of said parameter during each successive different predetermined time period, and a plurality of sets of values of administration of said pharmacologically active agent, each set comprising a plurality of administration values during each successive different predetermined time period;
a display controller, for controlling the display (60) to display the measured values of the parameter, the values of administrations of said pharmacologically active agent, and the visual link in accordance with the method of any one of claims 1 to 8.

10. A computer program comprising program code means for executing on an electronic device a method in accordance with any one of claims 1 to 8.

11. A computer program according to claim 10 wherein the electronic device is a mobile telephone, PDA or other portable electronic device.

12. A mobile telephone, PDA or other portable electronic device having loaded thereon an executable application for executing on the device a method according to any one of claims 1 to 8.

## Patentansprüche

1. Verfahren zum Anzeigen von Daten, die auf die Kontrolle eines Gesundheitszustands über mehrere aufeinander folgende unterschiedliche Zeiträume durch Verabreichung eines pharmakologischen Wirkstoffs hindeuten, welches umfasst:
Erfassen von mehreren Sätzen von Messwerten eines Parameters, die auf den Gesundheitszustand hindeuten, wobei jeder Satz mehrere Messwerte des Parameters während jedes aufeinander folgenden unterschiedlichen vorbestimmten Zeitraums umfasst, und von mehreren Sätzen von Verabreichungswerten des pharmakologischen Wirkstoffs, wobei jeder Satz mehrere Verabreichungswerte während jedes aufeinander folgenden unterschiedlichen vorbestimmten Zeitraums umfasst, als Daten;
Anzeigen einer Darstellung der Messwerte des Parameters aus allen Sätzen gegen eine Zeitachse in einer ersten Anzeigefläche (61);
Anzeigen einer zeitlichen Darstellung der Werte der Verabreichungen des pharmakologischen Wirkstoffs aus allen Sätzen gegen eine Zeitachse in einer zweiten, benachbarten Anzeigefläche (62);
wobei die erste und die zweite Anzeigefläche (61, 62) jeweils einen einzelnen repräsentativen Zeitraum, der von der gleichen Länge wie einer der aufeinander folgenden unterschiedlichen vorbestimmten Zeiträume ist, anzeigen, wobei alle Verabreichungs- und Messwerte aus den aufeinander folgenden unterschiedlichen Zeiträumen in dem einzigen repräsentativen Zeitraum dargestellt sind; und
Anzeigen einer visuellen Verbindung zwischen jedem Paar angezeigter Werte, das durch einen Verabreichungswert und einen Messwert des Parameters gebildet ist, der der Reaktion des Gesundheitszustands auf die Verabreichung entspricht; und wobei
die visuelle Verbindung: (a) entsprechenden Paaren aus jedem der Sätze gemeinsam ist; und (b) die unterschiedlichen Paare in jedem Satz visuell voneinander unterscheidet.

2. Verfahren nach Anspruch 1, **dadurch gekennzeichnet, dass** die visuelle Verbindung eine Farbkodierung der dargestellten Werte umfasst.

3. Verfahren nach Anspruch 1 oder 2, **dadurch gekennzeichnet, dass** die Zeitachsen in den zwei Anzeigeflächen ausgerichtet sind.

4. Verfahren nach Anspruch 1, 2 oder 3, **dadurch gekennzeichnet, dass** der vorbestimmte Zeitraum ein Tag ist.

5. Verfahren nach Anspruch 4, **dadurch gekennzeichnet, dass** die entsprechenden Paare aus jedem Satz Paare sind, die sich zeitlich entsprechen.

6. Verfahren nach Anspruch 4 oder 5, **dadurch gekennzeichnet, dass** die entsprechenden Paare aus jedem Satz Paare sind, die die gleiche Uhrzeit aufweisen.

7. Verfahren nach Anspruch 4 oder 5, **dadurch gekennzeichnet, dass** die entsprechenden Paare aus jedem Satz Paare sind, die während jedes der Tage den gleichen jeweiligen Tätigkeiten entsprechen.

8. Verfahren nach Anspruch 4 oder 5, **dadurch gekennzeichnet, dass** die entsprechenden Paare aus jedem Satz Paare sind, die während jedes der Tage den gleichen Mahlzeiten und der Schlafenszeit entsprechen.

9. Vorrichtung zum Anzeigen von Daten, die auf die Kontrolle eines Gesundheitszustand über mehrere aufeinander folgende unterschiedliche Zeiträume durch Verabreichung eines pharmakologischen Wirkstoffs hindeuten, welche umfasst:
eine Anzeige (60);
eine Datenerfassungsvorrichtung zum Erfassen von mehreren Sätzen von Messwerten eines Parameters, die auf den Gesundheitszustand hindeuten, wobei jeder Satz mehrere Messwerte des Parameters während jedes aufeinander folgenden unterschiedlichen vorbestimmten Zeitraums umfasst, und von mehreren Sätzen von Verabreichungswerten des pharmakologischen Wirkstoffs, wobei jeder Satz mehrere Verabreichungswerte während jedes aufeinander folgenden unterschiedlichen vorbestimmten Zeitraums umfasst, als Daten;
ein Anzeigesteuergerät zum Steuern der Anzeige (60), um die Messwerte des Parameters, die Verabreichungswerte des pharmakologischen Wirkstoffs und die visuelle Verbindung gemäß dem Verfahren nach einem der Ansprüche 1 bis 8 anzuzeigen.

10. Computerprogramm, das Programmcode-Mittel zum Ausführen eines Verfahrens nach einem der Ansprüche 1 bis 8 auf einer elektronischen Vorrichtung umfasst.

11. Computerprogramm nach Anspruch 10, **dadurch gekennzeichnet, dass** die elektronische Vorrichtung ein Mobiltelefon, ein PDA oder eine andere tragbare elektronische Vorrichtung ist.

12. Mobiltelefon, PDA oder andere tragbare elektronische Vorrichtung mit einer darauf geladenen ausführbaren Anwendung zum Ausführen eines Verfahrens nach einem der Ansprüche 1 bis 8 auf der Vorrichtung.

## Revendications

1. Procédé d'affichage de données indicatives de la commande de conditions médicales sur plusieurs périodes de temps différentes successives par administration d'un agent actif d'un point de vue pharmacologique, comportant les étapes consistant à :
acquérir en tant que lesdites données une pluralité d'ensembles de valeurs mesurées d'un paramètre indicatif de ladite condition médicale, chaque ensemble comportant une pluralité de valeurs mesurées et dudit paramètre pendant chaque période de temps prédéterminée différente successive, et une pluralité d'ensembles de valeurs d'administration dudit agent actif d'un point de vue pharmacologique, chaque ensemble comportant une pluralité de valeurs d'administration pendant chaque période de temps prédéterminée différente successive,
afficher dans une première zone d'affichage (61) un tracé des valeurs mesurées du paramètre de tous lesdits ensemble par rapport à un axe de temps,
afficher dans une seconde zone d'affichage (62) adjacente un tracé temporel des valeurs d'administrations dudit agent actif d'un point de vue pharmacologique de tous lesdits ensemble par rapport un axe de temps,
chacune desdites première et seconde zones d'affichage (61, 62) affichant une période de temps représentative unique égale en longueur à l'une desdites périodes de temps prédéterminées différentes successives, l'ensemble des valeurs d'administration et mesurées à partir desdites périodes de temps différentes successives étant tracées dans ladite période de temps représentative unique, et
afficher une liaison visuelle entre chaque paire de valeurs affichées formées d'une valeur d'administration et d'une valeur mesurée du paramètre correspondant à une réponse de la condition médicale à cette administration, et dans lequel
ladite liaison visuelle est : (a) commune pour des paires correspondantes de chacun des ensembles, et (b) distingue visuellement les différentes paires les unes des autres dans chaque ensemble.

2. Procédé selon la revendication 1, dans lequel la liaison visuelle comporte un codage de couleurs desdites valeurs tracées.

3. Procédé selon la revendication 1 ou 2, dans lequel les axes de temps dans les deux zones d'affichage sont alignés.

4. Procédé selon la revendication 1, 2 ou 3, dans lequel la période de temps prédéterminée est un jour.

5. Procédé selon la revendication 4, dans lequel les paires correspondantes de chaque ensemble sont des paires qui correspondent d'un point de vue temporel.

6. Procédé selon la revendication 4 ou 5, dans lequel les paires correspondantes de chaque ensemble sont des paires qui sont à la même heure du jour.

7. Procédé selon la revendication 4 ou 5, dans lequel les paires correspondantes de chaque ensemble sont des paires qui correspondent aux mêmes activités respectives pendant chacun des jours.

8. Procédé selon la revendication 4 ou 5, dans lequel les paires correspondantes de chaque ensemble sont des paires qui correspondent aux mêmes repas et aux mêmes heures de coucher pendant chacun des jours.

9. Dispositif pour afficher des données indicatives de la commande d'une condition médicale sur plusieurs périodes de temps différentes successives par administration d'un agent actif d'un point de vue pharmacologique, comportant :
un écran (60),
un dispositif d'acquisition de données pour acquérir en tant que lesdites données une pluralité d'ensembles de valeurs mesurées d'un paramètre indicatif de ladite condition médicale, chaque ensemble comportant une pluralité de valeurs mesurées dudit paramètre pendant chaque période de temps prédéterminée différente successive, et une pluralité d'ensembles de valeurs d'administration dudit agent actif d'un point de vue pharmacologique, chaque ensemble comportant une pluralité de valeurs d'administration pendant chaque période de temps prédéterminée différente successive,
un dispositif de commande d'affichage pour commander l'affichage (60) afin d'afficher les valeurs mesurées du paramètre, les valeurs d'administrations dudit agent actif d'un point de vue pharmacologique, et la liaison visuelle conformément au procédé selon l'une quelconque des revendications 1 à 8.

10. Programme informatique comportant des moyens de code de programme pour mettre en oeuvre sur un dispositif électronique un procédé conformément à l'une quelconque des revendications 1 à 8.

11. Programme informatique selon la revendication 10, dans lequel le dispositif électronique est un téléphone mobile, un assistant numérique personnel ou un autre dispositif électronique portable.

12. Téléphone mobile, assistant numérique personnel ou autre dispositif électronique portable ayant chargé sur celui-ci une application exécutable pour mettre en oeuvre sur le dispositif un procédé conformément à l'une quelconque des revendications 1 à 8.
